# EUROPEAN PATENT APPLICATION

(11) **EP 4 434 430 A1**
(43) Date of publication of application: **25.09.2024**
(21) Application number: 23164120.0
(22) Date of filing: 24.03.2023
(51) Int. Cl.: A61B 1/00, A61B 1/12, A61B 90/70, B08B 9/00

(54) **A CHANNEL IRRIGATION SYSTEM FOR FLUSHING PROCESS MEDIUM THROUGH INTERNAL CHANNELS OF A MEDICAL ARTICLE**

(71) Applicant: Getinge Disinfection AB, 351 15 Växjö (SE)
(72) Inventor: LAGERKVIST, Carl-Johan, 351 15 Växjö (SE)
(74) Representative: Zacco Sweden AB

(57) **Abstract**

A channel irrigation system (10) for flushing process medium through internal channels of a medical article, in particular of an endoscope. A flow producing device (12) produces and supplies a flow of a process medium to a manifold (14). A plurality of fluid passages (24, 26) extend from respective outlets of the manifold. Each fluid passage has another end connectable to a respective internal channel of a medical article to be flushed with the process medium. A pressure sensor (28) measures the current pressure of the process medium inside the manifold. A control unit (100) receives from the pressure sensor information about the value of the current pressure and, based on the received information, controls the operation of the flow producing device so that the process medium inside the manifold reaches a target pressure or target pressure range.

## Description

### TECHNICAL FIELD

The present disclosure relates to a channel irrigation system for flushing process medium through internal channels of a medical article, in particular of an endoscope. The present disclosure also relates to an endoscope washer-disinfector comprising such a channel irrigation system.

### BACKGROUND ART

Medical articles that have multiple channels are frequently used in hospitals, such as in surgical departments and similar facilities. The channels may be used for providing access to body organs or internal body cavities without any, or with only limited, surgical intervention. For instance, different types of endoscopes are examples of such medical articles.

Such a medical article should be washed/disinfected after it has been used, so that it can be re-used at another occasion. Washing and/or disinfecting may for example be performed in combined medical washer-disinfectors that are typically provided at surgical departments, central sterile supply departments, and similar facilities. The medical washer-disinfectors are configured to allow the medical articles to be washed on the outside as well internally, i.e. inside the channels. In particular, the medical washer-disinfectors may include a channel irrigation system for providing a process medium to the inside of the channels of the medical articles. Process media such as water, disinfection chemicals, alcohol, and/or detergent can be supplied by the channel irrigation system to the respective channels. An appropriate flow of process medium is desired for satisfactory cleaning and disinfection. However, controlling the flow through the channels in a repeatable and accurate manner is challenging. High repeatability is desirable in order to allow for monitoring and controlling the flow through the channels against individual predefined flow limit values. The flow limit values may be established during qualification testing of each individual medical article or type of medical article. Further complexity is added when, for instance, different endoscope types are taken into account. Influencing factors that may vary are the number of channels, their diameter, and their length. If the flow is too low or too high, the process could be automatically stopped and an alarm may be generated. Thus, due to lack of precision in controlling the flow, false alarms may sometimes be triggered, causing unnecessary interruption of the process.

### SUMMARY OF THE DISCLOURE

An object of the present disclosure is to mitigate drawbacks of conventional channel irrigation systems, such as inconsistent flow rates, difficulty controlling flow rates into channels, and the need to use more costly flow rate monitors. This and other objects, which will become apparent in the following, are accomplished by a channel irrigation system as defined in claim 1. Some non-limiting exemplary embodiments are presented in the dependent claims.

The disclosure includes the realization that by continuously measuring the current pressure of the process medium and controlling, based on the measurement, a flow producing device to produce a flow which reaches a target pressure or target pressure range, a superior process control can be achieved when cleaning medical articles having internal channels. In particular, it has been realized that by being able to control the flow producing device to reach a target pressure, even low-cost flow sensors such as a paddlewheel flow sensor (which normally require a minimum working pressure to give accurate readings) may be used for measuring the flow to the channels. This provides good control of the cleaning process at a reduced cost compared to existing systems.

According to at least a first aspect of the present disclosure, there is provided a channel irrigation system for flushing process medium through internal channels of a medical article, in particular of an endoscope, comprising:
- a flow producing device configured to produce and supply a flow of a process medium,
- a manifold having an inlet and a plurality of outlets, the inlet being configured to receive the process medium supplied from the flow producing device,
- a plurality of fluid passages, each fluid passage having a first end connected to a respective one of said plurality of outlets for receiving process medium from the manifold, each fluid passage having a second end connectable to a respective internal channel of a medical article to be flushed with the process medium,
- a pressure sensor configured to measure the current pressure of the process medium inside the manifold, in particular a pressure sensor in fluid communication with the inside of the manifold, and
- a control unit configured to receive from the pressure sensor information about the value of the current pressure and, based on the received information, control the operation of the flow producing device so that the process medium inside the manifold reaches a target pressure or target pressure range.

By controlling the pressure of the process medium inside the manifold to reach a target pressure or target pressure range, an accurate flow control to the internal channels of the medical article is possible. In particular, accurate flow control is possible even with relatively low-cost flow sensors. Many flow sensors only operate accurately within a certain pressure range and they need a minimum starting pressure of the process medium to get any reading at all. By controlling the pressure within the manifold, inaccurate readings of flow sensors may be avoided, and the overall process control may be improved.

The channel irrigation system may be used for washing (cleaning) and/or disinfecting the internal channels of medical articles. It should be understood that in this disclosure, the terms "washing" and "cleaning" are considered interchangeable and that the channel irrigation system is for both.

As indicated above, the channel irrigation system may be particularly suitable for cleaning endoscopes. It should, however, by understood that the channel irrigation system may also be used for cleaning other medical articles having internal channels. For instance, medical instruments, such as some instruments used in robotic surgery, may have long internal channels which may suitably be cleaned by means of the channel irrigation system of the present disclosure.

Once said target pressure or said pressure range has been reached, it may be desirable to maintain the pressure substantially constant in order to maintain high accuracy of the process control. This is reflected in at least one exemplary embodiment, according to which, upon determination by the control unit that the current pressure corresponds to said target pressure or target pressure range, the control unit is configured to control the operation of the flow producing device so that the pressure of the process medium inside the manifold is maintained at said target pressure or within said target pressure range. By maintaining the pressure within the manifold above the target pressure or target pressure range, in particular by maintaining a substantially constant pressure, a predictable and accurate cleaning of the internal channels may be achieved. Avoiding undesirable pressure variations and thus undesirable flow rate variations provides for a reliable and robust processing of the internal channels of the medical article.

According to at least some exemplary embodiments, said controlling of the operation of the fluid processing device includes the control unit controlling the speed of the flow producing device. The flow producing device may, for instance, be a motor-driven pump. By receiving the feedback from the pressure sensor, the control unit can adjust the speed until the pressure sensor indicates that a desired pressure has been reached inside the manifold. Thus, during start-up, the control unit will increase the speed of the flow producing device until sufficient pressure has been built up in the manifold.

It has been found particularly advantageous to use a gear pump as said flow producing device. The hydraulic characteristics of gear pumps are highly beneficial for the operation of the channel irrigation system of this disclosure. In particular, gear pumps produce a linear flow relative to the speed of the pump. Thus, the speed of the gear pump is normally directly translatable to the flow rate produced by the gear pump, and thus also to the pressure. In contrast, other commonly used pumps in washer-disinfectors, in particular centrifugal pumps, do not have such linear performance in terms of rotational speed versus flow. Accordingly, in exemplary embodiments of this disclosure, wherein the flow producing device is a gear pump, it becomes easy to control the flow and the pressure of the process medium with high precision. When flow and pressure are accurately controlled, a high repeatability and improved flow control to the internal channels of the medical article is achieved. Any flow sensors work better under conditions when the pressure can be controlled and maintained within a target pressure range.

In as least some exemplary embodiments, by having an accurate pressure control, as discussed above, in particular by using a gear pump as said flow producing device, an accurate flow control is achieved without requiring any flow sensors. By knowing the required flow for the individual medical article to be treated, the control unit may simply set the speed of the gear pump to reach the desired flow rate. Nevertheless, as a safety measure, it may be advisable to also incorporate one or more flow sensors in order to be able to detect any disturbance causing the real flow to deviate from the expected flow. Such disturbances may, for instance, be caused by clogging of one or more internal channels.

Thus, in some embodiments of this disclosure the channel irrigation system may comprise one or more flow sensors. Flow sensors may advantageously be provided for detecting errors in the cleaning process. For instance, if a flow sensor detects a lower flow than what would be expected through one of the internal channels of the medical article, then this may be indicative of the internal channel being partly clogged. By having accurate pressure control as discussed above, the readings of the flow sensor can be relied upon, and an alert/alarm may be set off when appropriate.

According to at least one exemplary embodiment, the channel irrigation system comprises:
- a conduit provided between the flow producing device and the manifold,
- an upstream flow sensor configured to measure the current flow rate in said conduit, and
- a plurality of downstream flow sensors, each downstream flow sensor being configured to measure the current flow rate in a respective one of said plurality of fluid passages.

By measuring the flow upstream as well as downstream of the manifold various anomalies may be detected. For instance, expected nominal values upstream and downstream of the manifold may be compared to measured values. For instance, if one of the downstream flow sensors measure a flow rate which differs by a certain amount, e.g. being 10% outside the nominal flow rate, then this may be indicative of an error, such as an obstruction in that flow passage or in the internal channel connected to that flow passage. Another example is detection of anomalies with respect to the flow through the manifold. The total flow rate downstream of the manifold should be substantially equal to the flow rate upstream of the manifold. Thus, the sum of the individual flow rates (typically volume per unit of time) measured by the plurality of downstream flow sensors should be substantially equal to the flow rate measured by the upstream flow sensor. The control unit may suitably be configured to receive input information from each one of the flow sensors and upon detection of any irregularity, such as the flow rate from an individual flow sensor does not sense the expected flow rate, or that the total flow rate downstream of the manifold differs from the flow rate upstream of the manifold by a predefined amount (e.g. by an absolute amount or by a relative amount), then the control unit may trigger an alarm or other type of notification in order to alert the relevant personnel. The latter situation is at least partly reflected in the following exemplary embodiment.

Thus, according to at least one exemplary embodiment, the control unit is configured to receive from the upstream flow sensor and the plurality of downstream flow sensors information about the value of the current flow rate in the conduit and the plurality of fluid passages, respectively,
wherein, upon determination by the control unit that the sum of the current flow rates of the plurality of fluid passages differs from the current flow rate in the conduit by at least a predefined amount, the control unit is configured to trigger an alert or alarm.

Hereby, malfunctioning of one or more of the downstream flow sensors may be detected. The upstream flow sensor may be provided to double-check the functioning of the downstream flow sensors. If the sum of the flow rates presented by the downstream flow sensors do not add up to the flow rate presented by the upstream flow sensor, this may be indicative of one or more of the downstream flow sensors not functioning properly, and thus not providing an accurate reading of the flow rate in the respective fluid passage.

As understood from the above discussion, according to at least one exemplary embodiment, for each one of said plurality of fluid passages, the control unit is configured to compare the value of the current flow rate with a respective nominal value,
wherein, upon determination by the control unit that the value of the current flow rate in any one of said plurality of fluid passages, deviates from its respective nominal value by at least a predetermined amount, the control unit is configured to trigger an alert or alarm.

As mentioned previously, by providing the channel irrigation system with the pressure control/feedback, the readings of the flow sensor can be properly relied upon, even for less costly flow sensors which are normally limited to a certain pressure range for accurate functioning. Considering the fact that medical articles, such as endoscopes, include multiple internal channels, the provision of a respective flow sensor for each internal channel may amount to a considerable investment depending on the type of flow sensor selected. Thus, providing a pressure control which allows for less costly flow sensors to be used is indeed beneficial. One such type of flow sensor will now be briefly discussed.

According to at least one exemplary embodiment, the channel irrigation system comprises at least one paddlewheel flow sensor configured to measure a current flow rate of the process medium. Paddlewheel flow sensors have the advantage of being low cost, however, they normally require a certain working pressure for good accuracy. Therefore, in combination with the pressure control of this disclosure, in particular when using a gear pump having linear characteristics as discussed previously, using one or more paddlewheel flow sensors allows for accurate yet cost-efficient flow measurements.

According to at least one exemplary embodiment, at least one of said upstream flow sensor and said plurality of downstream flow sensors is in the form of a paddlewheel sensor. Suitably, each one of said upstream flow sensor and said plurality of downstream flow sensors may be in the form of a paddlewheel flow sensor. When it is desired to provide multiple flow sensors in order to allow for individual flow measurements with respect to the plurality of internal channels of the medical articles to be processed, the provision of a paddlewheel flow sensors is particularly beneficial from an economic perspective, in particular since the functionality of such flow sensors is reliable in connection with the pressure control of the present disclosure.

According to at least one exemplary embodiment, the channel irrigation system further comprises an alcohol injector configured to inject alcohol into the manifold to be further distributed into said plurality of fluid passages via said outlets of the manifold. Alcohol injection is beneficial as it aids the drying of the internal channels of the medical article. Suitably, alcohol may be applied after the final rinse stage. Furthermore, before the alcohol is injected, compressed air may be injected into the manifold in order to remove larger amounts of residual water from the system. Subsequently, alcohol may suitably be injected together with compressed air. By injecting the alcohol together with compressed air, the compressed air assists in faster distribution of the alcohol in the internal channels of the medical article. The control unit may suitably be configured to control the supply of compressed air, e.g. by controlling the opening/closing of a pressurized tank outlet, and/or by controlling the operation of an air compressor.

According to at least one exemplary embodiment, the control unit is configured to control the execution of one or more initial treatment stages in which the flow producing device is controlled to provide the process medium to the plurality of fluid passages via the manifold, and subsequently control the execution of one or more final treatment stages in which the alcohol injector is controlled to inject alcohol into the manifold. There may be different process media provided during different treatment stages. Generally, the process medium may include a liquid, such as water, with or without additives. For instance, in one or more pre-rinse stage the process medium may be just water, while in a subsequent wash stage the process medium may include water mixed with detergent and/or disinfectant. The channel irrigation system may suitably also include post and/or final rinse stages in order to flush away detergents. The alcohol injection may suitably be performed after such a final rinse stage in order to dry the internal channels of the medical article (with or without the assistance of compressed air as discussed above).

According to at least one exemplary embodiment, said pressure sensor is a first pressure sensor, wherein the channel irrigation system further comprises a redundancy pressure sensor configured to measure the current pressure of the process medium inside the manifold, wherein upon determination by the control unit that the respective values of the current pressure provided by the first pressure sensor and the redundancy pressure sensor deviate from each other by at least a predefined amount, the control unit is configured to issue an alert or alarm. A technical benefit of providing a redundancy pressure sensor is that, in case of failure of the first pressure sensor, this will be noticed in time, and incorrect process control may be avoided.

According to at least one exemplary embodiment, in particular for simultaneously flushing process medium through endoscope channels having different widths, said flow producing device is a first flow producing device, said manifold is a first manifold, said plurality of fluid passages is a first plurality of fluid passages, and said pressure sensor is a first pressure sensor, the channel irrigation system further comprising:
- a second flow producing device configured to produce and supply a flow of a process medium,
- a second manifold having an inlet and a plurality of outlets, the inlet being configured to receive the process medium supplied from the second flow producing device,
- a second plurality of fluid passages, each fluid passage having a first end connected to a respective one of said plurality of outlets of the second manifold for receiving process medium from the second manifold, each fluid passage having a second end connectable to a respective internal channel of a medical article to be flushed with the process medium,
- a second pressure sensor configured to measure the current pressure of the process medium inside the second manifold,
wherein the control unit is configured to receive from the second pressure sensor information about the value of the current pressure and, based on the received information, control the operation of the second flow producing device so that the process medium inside the second manifold reaches a target pressure or target pressure range.

This provides more flexibility as different pressures and flow rates may be provided to different channels. Furthermore, by having the above exemplified dual set of flow producing devices and manifolds, etc., a higher accuracy may be achieved. For instance, for some medical articles, such as for certain types of endoscopes, some internal channels may be wider than others. The first plurality of fluid passages may, for example, be connected to wider internal channels, while the second plurality of fluid passages may be connected to narrower channels. In this manner, the risk of pressure drops in one channel negatively affecting the flushing in another channel may be reduced. In particular, an individual endoscope may have one or more relatively large (wide) channels as well as one or more relatively small (narrow) channels. In such case, the individual endoscope may be connected to both the first and the second plurality of passages (for example connecting the relatively large channel(s) to the first plurality of passages and connecting the relatively small channel(s) to the second plurality of passages). A technical benefit of this may be understood from the following illustrative example: Imagine first that you have an endoscope with two channels of identical width. They are connected to two equal fluid passages extending form the manifold. Since the channels of the endoscope are identical the flow of fluid will be equally distributed in the two channels. Next imagine that you still have the same two fluid passages extending from the manifold, but now you replace the previously connected endoscope which another endoscope having one extremely wide channel and one extremely narrow channel. In this latter case there will be less resistance for the fluid to flow through the extremely wide channel. The fluid will "prefer" to go into the extremely wide channel, but will of course also go into the narrow channel. Because of the size difference, however, it will be difficult to make sure that you simultaneously achieve the desired respective flow rate through both the extremely wide channel and the extremely narrow channel. Therefore, by allowing a grouping of endoscope channels into one group of relatively narrow channels and one group of relatively wide channels, each being connected to a respective manifold and its fluid passages, the risk of the differently sized channels negatively affecting each other will be reduced.

The lengths and width of the first and second plurality of fluid passages may be the same or they may be different from each other. For example, in some embodiments the first plurality of fluid passages may each have a larger diameter than the second plurality of fluid passages. Similarly to providing a redundancy pressure sensor associated with the first pressure sensor and the first manifold, there may suitably be provided another redundancy sensor associated with the second pressure sensor and the second manifold.

This disclosure includes electronics and electronic instructions for implementing the devices and processes described herein, which may be embodied and/or collectively described as a control unit. The control unit may include a microprocessor, microcontroller, programmable digital signal processor, computer, or another programmable device. The control unit may also, or instead, include an application specific integrated circuit, a programmable gate array or programmable array logic, a programmable logic device, or a digital signal processor. Where it includes a programmable device such as the microprocessor, microcontroller or programmable digital signal processor mentioned above, the processor may further include computer executable code that controls operation of the programmable device as disclosed herein. The control unit is preferably configured for use with medical washer-disinfectors, in particular endocope reprocessor, and provided with electronic instructions to perform and control the flushing of process medium through internal channels of medical articles.

According to at least a second aspect of the present disclosure, there is provided an endoscope washer-disinfector comprising the channel irrigation system according to the first aspect, including any exemplary embodiment thereof, the endoscope washer-disinfector being configured for flushing the process medium through internal channels of one or more endoscopes using the channel irrigation system for cleaning them.

The person of skill in this technology will understand that the various features and exemplary embodiments discussed in connection with the channel irrigation system of the first aspect may be readily implemented as corresponding exemplary embodiments in the endoscope washer-disinfector of the second aspect. The advantages of the endoscope washer-disinfector of the second aspect largely corresponds to the advantages of the channel irrigation system of the first aspect, including any exemplary embodiments thereof.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order disclosed, unless explicitly stated. Further features of, and advantages with, the present disclosure will become apparent when studying the appended claims and the following description. The skilled person realizes that different features of the present disclosure may be combined to create embodiments other than those described in the following, without departing from the scope of the present disclosure.

### BRIEF DESCRIPTION OF THE DRAWINGS

Fig. 1 is a perspective view of one type of washer-disinfector in which a channel irrigation system of the present disclosure may be implemented.
Fig. 2 is a schematic illustration of a channel irrigation system according to at least one exemplary embodiment of the present disclosure.
Fig. 3 is a schematic illustration of some details of a channel irrigation system according to at least one exemplary embodiment of the present disclosure.
Fig. 4 is a schematic illustration of a gear pump that may be included in a channel irrigation system according to at least one exemplary embodiment of the present disclosure.
Fig. 5 is a schematic illustration of a channel irrigation system according to at least another exemplary embodiment of the present disclosure.

### DETAILED DESCRIPTION

The present disclosure will now be described more fully hereinafter with reference to the accompanying drawings, in which certain aspects of a channel irrigation system of the present disclosure are shown. The channel irrigation system may, however, be embodied in many different forms and should not be construed as limited to the embodiments and aspects set forth herein; rather, the embodiments are provided by way of example so that this disclosure will be thorough and complete, and will fully convey the scope of the channel irrigation system to those skilled in the art. Accordingly, it is to be understood that the present disclosure is not limited to the embodiments described herein and illustrated in the drawings; rather, the skilled person will recognize that many changes and modifications may be made within the scope of the appended claims. Like reference numerals refer to like elements throughout the description.

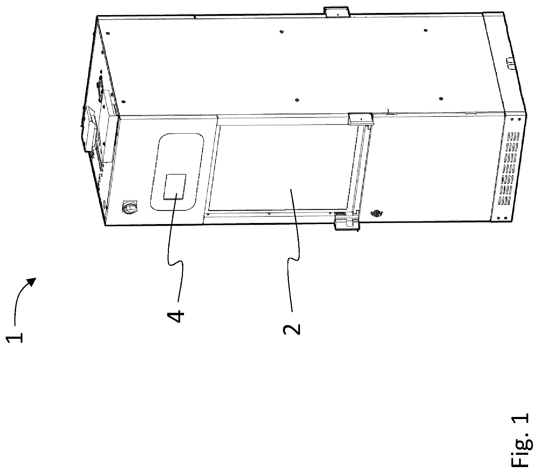

Fig. 1 is a perspective view of one type of washer-disinfector 1 in which a channel irrigation system of the present disclosure may be implemented. The washer-disinfector 1 may in particular be in the form of an endoscope washer-disinfector configured for flushing process medium through internal channels of one or more endoscopes using the channel irrigation system of this disclosure. The washer-disinfector comprises a washing chamber for receiving medical articles, such as endoscopes. The washing chamber can be closed by means of a slidable door 2. The door is shown in its closed position, and therefore the washing chamber is not visible in the drawing. The door 2 may suitably be slid downwardly in order to provide access to the washing chamber. The washer-disinfector 1 may thus be used for washing (cleaning) and/or disinfecting medical articles located in the washing chamber. The washer-disinfector 1 may also after such washing and/or disinfecting be used for drying the medical articles.

Although the present disclosure is focused on how to process the internal channels of the medical articles, it should be understood that the washer-disinfector 1 may suitably also be used for cleaning the external surfaces of the medical article. Thus, the washer-disinfector 1 may suitably, in addition to the channel irrigation system of this disclosure, comprise nozzles or other types of outlets that are used for providing process medium externally of the medical articles. Such nozzles or outlets may, for instance, be provided on one or more spray wings, which may, for instance, be mounted on a side wall, a bottom wall or a top wall defining the washing chamber.

A user interface 4, such as a touch control panel, may be provided for displaying information and enabling a user to select a program. Some examples of information that may be presented at the user interface 4 include remaining processing time (such as total time or relating to a currently performed treatment stage), an identification of the medical article or articles that are present in the washing chamber, any notification to the personnel, such as an alert or alarm indicative of an error, anomaly, etc.

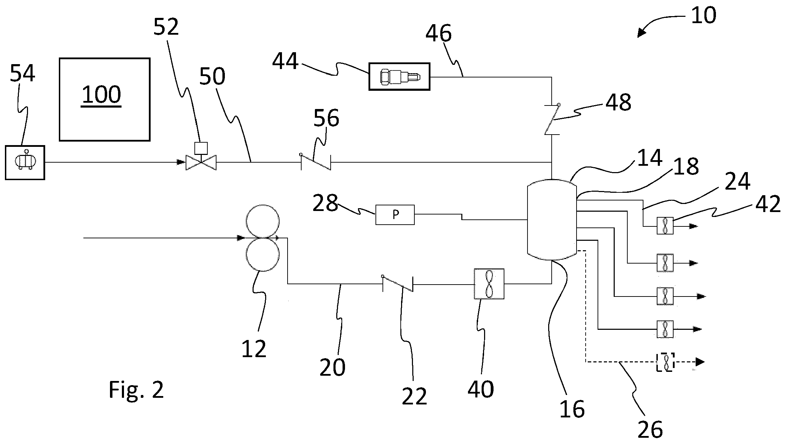

Fig. 2 is a schematic illustration of a channel irrigation system 10 according to at least one exemplary embodiment of the present disclosure. The channel irrigation system 10 may, for instance, be included in the washer-disinfector 1 illustrated in Fig. 1, or in any other suitable washer-disinfector. In particular, the channel irrigation system 10 may be included in a medical washer-disinfector, such as an endoscope washer-disinfector.

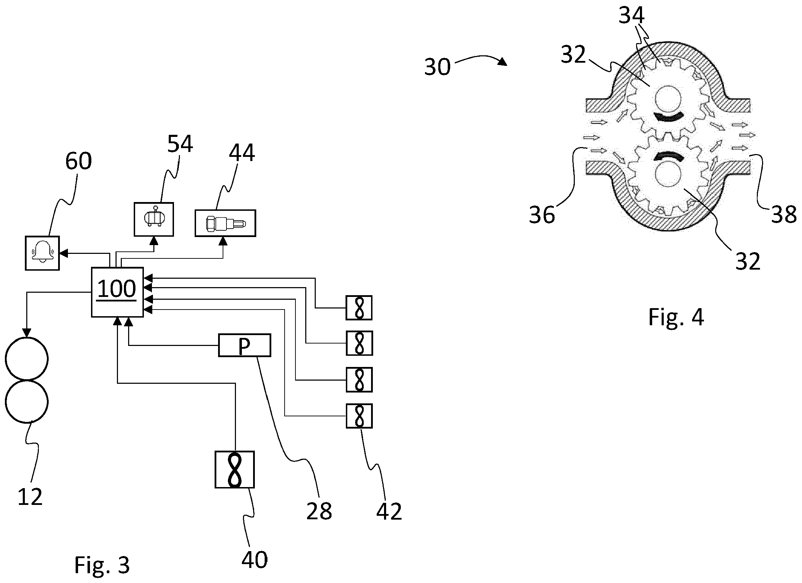

The channel irrigation system 10 is provided for flushing process medium through internal channels of a medical article, such as an endoscope, robotic medical instruments, etc. The channel irrigation system 10 is particularly suitable for providing treatment to a medical article having multiple internal channels. The channel irrigation system 10 comprises at least one flow producing device 12 which is configured to produce and supply a flow of a process medium. The flow producing device 12 may suitably be a gear pump, such as the one schematically illustrated in Fig. 4, which will later be discussed in more detail.

The channel irrigation system 10 further comprises a manifold 14 having an inlet 16 and a plurality of outlets 18. The inlet 16 is configured to receive the process medium supplied from the flow producing device 12. As illustrated in Fig. 2 a conduit 20 may extend from the flow producing device 12 to the inlet 16 of the manifold 14. The process medium may pass through the conduit 20 to the manifold 14. The flow producing device 12 may suitably pump process medium (such as water, with or without additives) from a source or reservoir containing the process medium (not illustrated). A check valve 22 may be provided in the conduit 20 between the flow producing device 12 and the manifold 14 in order to avoid any backflow through the conduit 20 when the flow producing device 12 is not in operation.

The channel irrigation system 10 further comprises a plurality of fluid passages 24, 26. Each fluid passage 24, 26 has a first end connected to a respective one of said plurality of outlets 18 for receiving process medium from the manifold 14. Each fluid passage 24, 26 also has a second end connectable to a respective internal channel of a medical article to be flushed with the process medium. The second ends may either be directly connectable to the internal channels or via one or more intermediate connecting devices. In some exemplary embodiments, the second ends of the fluid passages 24, 26 may suitably be formed by male connectors configured to be engaged with female connectors associated with the internal channels of the medical article or associated with any intermediate connecting device. In some exemplary embodiments, the second ends of the fluid passages may suitably be formed by female connectors configured to be engaged with male connectors associated with the internal channels or associated with any intermediate connecting device.

In Fig. 2, four (24) fluid plus one (26) fluid passages are illustrated. The fifth fluid passage 26 is symbolically illustrated by a dashed line, representing that the number of fluid passages may be more than the five illustrated. For example, there may be six, seven, eight or more fluid passages. Although it may be of limited use in practical implementations, it should be understood that the present disclosure is not limited to a channel irrigation system having four or more fluid passages. The technical concepts of this disclosure may be implemented in a channel irrigation system having fewer than four fluid passages, which is therefore also encompassed by the present disclosure.

The channel irrigation system 10 further comprises a pressure sensor 28 configured to measure the current pressure of the process medium inside the manifold 14. Thus, the pressure sensor 28 may suitably be in fluid communication with the process medium inside the manifold 14, such that the pressure sensor 28 is subjected to the same fluid pressure as the manifold 14.

A control unit 100 has been schematically illustrated and forms part of the channel irrigation system 10. The control unit 100 may suitably control the operation of the channel irrigation system 10 as such. However, in at least some exemplary embodiment, the control unit 100 may also control the operation of other parts of the washer-disinfector in which the channel irrigation system 10 is provided.

In order to not obscure the legibility of the drawings, the control unit 100 has been illustrated as a separate component. It should, however, be understood that the control unit 100 may be appropriately operatively connected to other components of the channel irrigation system 10, and suitably to other components of the overall washer-disinfector, such as by wired or by wireless communication.

The control unit 100 is in particular configured to receive from the pressure sensor 28 information about the value of the current pressure inside the manifold 14. Based on the received information, the control unit 100 may control the operation of the flow producing device 12 so that the process medium inside the manifold 14 reaches a target pressure or target pressure range. Since different medical articles will have different designs with respect to diameters and lengths of the internal channels, the control unit 100 may adapt its control of the operation of the flow producing device 12 based on the design of the medical articles to be processed.

As previously discussed, when the control unit 100 has determined that the current pressure corresponds to the target pressure or target pressure range, then the control unit 100 may control the operation of the flow producing device 12 so that the pressure of the process medium inside the manifold 14 is maintained at the target pressure or within the target pressure range. Thus, when the desired target pressure (or target pressure range) has been reached the control unit 100 continues to receive feedback from the pressure sensor 28 in order to properly control the flow producing device 12. After the target pressure (or target pressure range) has been reached, there may still be pressure-affecting changes. For instance, changes in water temperature may cause changes in pressure. Another example is when/if process chemicals are added to the water, which can also cause small changes. Thus, the control unit 100 may continue controlling the flow producing device 12 to counteract any such pressure-affecting changes.

The control unit 100 may suitably control the operation of the flow producing device 12 by controlling the speed of the flow producing device 12. In the case of the flow producing device 12 being in the form of a pump, the control unit 100 may control the rotational speed of the pump. Such speed control may suitably be an indirect control, such as controlling the rotational speed of a motor, which in turn drives the pump or other type of flow producing unit.

According to at least some exemplary embodiments, the flow producing unit 12 may suitably be, or comprise, a gear pump. Fig. 4 is a schematic illustration of such a gear pump 30. The gear pump 30 has two rotatable gear wheels 32, each gear wheel 32 being provided with cogs 34 or teeth. The cogs 34 of one wheel 32 interlock with the cogs 34 of the other wheel 32 and when the gear wheels 32 rotate, the fluid is mechanically moved. Fluid drawn into the gear pump 32 at the inlet side 36 becomes enclosed within the cavities formed between the cogs 34, and becomes moved to the outlet side 38 where the fluid is discharged. The gear pump 30 delivers a smooth pulse-free flow which is proportional to the rotational speed of the gear wheels 32. This allows for easy and high-precision control of the flow and pressure of the process medium, which in turn allows for high repeatability and improved flow control to the internal channels of the medical article.

Turning back to Fig. 2, the channel irrigation system 10 may suitably comprise one or more flow sensors 40, 42. In Fig. 2 a plurality of flow sensors 40, 42 are illustrated. More specifically, the illustrated channel irrigation system 10 in Fig. 2 comprises an upstream flow sensor 40 configured to measure the current flow rate in the conduit 20 that extends from the flow producing device 12 to the manifold 14. The upstream flow sensor 40 is located upstream of the manifold 14, with respect to the general direction of movement of the fluid flow. The illustrated channel irrigation system 100 also comprises a plurality of downstream flow sensors 42, which are located downstream of the manifold 14 with respect to the general direction of movement of the fluid flow. Each downstream flow sensor 42 is configured to measure the current flow rate in a respective one of the plurality of fluid passages 24, 26.

The flow sensors 40, 42 may indicate any malfunctioning, or clogging, or other undesired or unexpected flow disturbance. Therefore, the flow sensors 40, 42 may be in operative communication (wired or wireless) with the control unit 100, providing the control unit 100 with information about the currently measured flow rate. Thus, the control unit 100 may be configured to receive from the upstream flow sensor 40 and the plurality of downstream flow sensors 42 information about the value of the current flow rate in the conduit 20 and in the plurality of fluid passages 24, 26, respectively. Upon determination by the control unit 100 that the sum of the current flow rates of the plurality of fluid passages 24, 26 differs from the current flow rate in the conduit 20 by at least a predefined amount, the control unit 100 is configured to trigger an alert or alarm. In other words, the flow rate into the manifold 14 should substantially correspond to the flow rate out of the manifold 14.

The control unit 100 may also be configured to compare the value of the current flow rate for each one of the fluid passages 24, 26 with a respective nominal value. Such a nominal value may be stored in an electronic memory integrated in or accessible by the control unit 100. The nominal value may depend on the size and type of the connected internal channel, the type and model of medical article, etc. Upon determination by the control unit 100 that the value of the current flow rate in any one of the fluid passages 24, 26 deviates from its respective nominal value by at least a predetermined amount, the control unit 100 may trigger an alert or alarm. In other words, if the fluid does not flow through one or more of the fluid passages 24, 26 at the flow rate that would have been expected, and thus presumably does not flow through the respective connected internal channel of the medical article as expected, then the personnel may be notified of this anomaly so that appropriate measures may be taken. The user may, for example, follow a trouble-shooting scheme, such as checking if any tube has become bent or disconnected, or if there is any clogging along the flow path, including the flow passages and any connections, such as external or internal connections of an endoscope.

One or more of the illustrated flow sensors 40, 42 may suitably be in the form of paddle wheel flow sensors. While paddle wheel flow sensors are very cost effective, they normally need a certain working pressure for good accuracy. Combing the provision of paddle wheel flow sensors with the provision of a gear pump is therefore particularly advantageous, as the flow and pressure control can be made accurate, without any pulsing in the fluid flow, which could lead to inaccurate flow measurements.

As illustrated in Fig. 2, the channel irrigation system 10 may further comprise an alcohol injector 44 configured to inject alcohol into the manifold 14 to be further distributed into the plurality of fluid passages 24, 26 via the outlets 18 of the manifold 14. The alcohol may be injected via an alcohol supply passage 46, which may suitably be provided with a check valve 48 to prevent fluid from flowing in the reverse direction in the alcohol supply passage 46. Additionally, the channel irrigation system 10 may comprise an air supply passage 50 through which compressed air may be passed through the manifold 14 and to the plurality of fluid passages 24, 26. An actuator-controlled valve 52 may be opened to allow compressed air from, for example, a pressurized tank 54, to be supplied through the air supply passage 50 to the manifold 14. Suitably, a check valve 56 is provided between the actuator-controlled valve 52 and the manifold 14 to prevent fluid from flowing in the reverse direction in the air supply passage 50. After a final rinse stage of a treatment process, compressed air may be supplied via the air supply passage 50 to the manifold 14 in order to remove larger amounts of residual water in the system. The alcohol may then be injected from the alcohol injector 44 together with compressed air to the accelerate the drying of the internal channels of the medical article. The compressed air assists in rapidly distributing the alcohol in the internal channels, and thus reduces the drying time. The control unit 100 may suitably control the operation of the alcohol injector 44. Also, the control unit 100 may suitably control the supply of compressed air (e.g. by controlling an actuator of the actuator-controlled valve 52 or by controlling the pressurized tank 54 (e.g. if a controllable valve is integrated in the pressurized tank 54 instead of having the illustrated intermediate actuator-controlled valve 52).

The control unit 100 is not limited to the above mentioned operation after the final rinse stage. On the contrary, the control unit 100 may suitably be involved in each stage of the processing (cleaning, washing, disinfecting, rinsing, etc.) of the internal channels of the medical article. For instance, the control unit 100 may be configured to control the execution of one or more initial treatment stages in which the flow producing device 12 is controlled to provide the process medium to the plurality of fluid passages 24, 26 via the manifold 14, and subsequently control the execution of one or more final treatment stages in which the alcohol injector 44 is controlled to inject alcohol (and/or the actuator-controlled valve 52 is controlled to supply compressed air) into the manifold 14. Fig. 3 can be regarded as an exemplary illustrative and very schematic summary of the functioning of the control unit 100 discussed so far.

Thus, Fig. 3 illustrates a schematic overview of some different interactions between the control unit 100 and other components of the channel irrigation system. It should be understood that this is just an illustrative example, and that the number of components and the types of components may differ between different embodiments.

Fig. 3 illustrates that the control unit 100 receives from the pressure sensor 28 information about the current pressure of the process medium inside the manifold. The control unit 100 receives from the upstream flow sensor 40 and from the downstream flow sensors 42 information about the current flow rate measured by the respective flow sensors 40, 42. Thus, the control unit 100 may be configured to receive input information about various physical parameters related to the operation of the channel irrigation system.

In terms of output from the control unit 100, the control unit 100 may be configured to send various different demands, requests, control signals, etc. For example, the control unit 100 is configured to control the operation of the flow producing device 12 so that the process medium reaches a target pressure or target pressure range. Such a target pressure or a target pressure range may be stored in an electronic memory integrated in the control unit 100 or accessible by the control unit 100. The control unit 100 may upon detection of any anomaly, disturbance or other event that calls for the working personnel to be notified, suitably issue an alert or alarm 60. Such an alert or alarm 60 may, for instance, be in the form of a visual and/or audible notification presented through an appropriate interface, lamp, loudspeaker, control panel etc. of the washer-disinfector. However, the control unit 100 may also be configured to issue the alarm 60 by sending a notification to a remote unit, such as a cell phone or remote computer. Furthermore, the control unit 100 may, depending on the cause for the alert or alarm, interrupt or modify the processing of the medical article. Finally, as illustrated in Fig. 3, and as previously discussed, the control unit 100 may control the operation of the alcohol injector 44 and the supply of compressed air, such as by controlling the pressurized tank 54 and/or an actuator-controlled valve associated thereto.

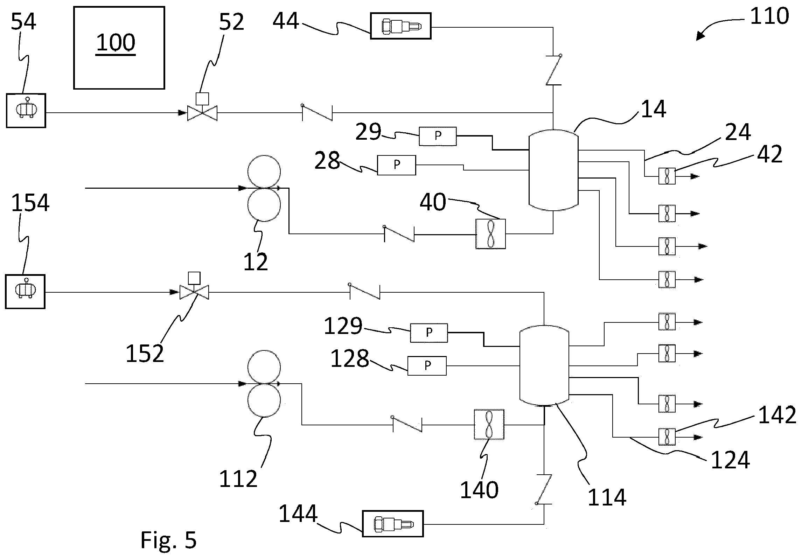

Fig. 5 is a schematic illustration of a channel irrigation system 110 according to at least another exemplary embodiment of the present disclosure. In comparison with the channel irrigation system 10 illustrated in Fig. 2, the channel irrigation system 110 illustrated in Fig. 5 has a double setup of the majority of the components. Instead of having all fluid passages and the connected internal channels being supplied with fluid from a common manifold, some of the internal channels will be supplied with fluid from a first manifold 14 while the other internal channels will be supplied with fluid from a second manifold 114. Hereby, different target pressures (or target pressure ranges) may be set for the first manifold 14 and the second manifold 114. As previously explained in this disclosure, this provides more flexibility as different pressures and flow rates may be provided to different channels. Furthermore, by having the a dual set of flow producing devices 12, 112 and manifolds 14, 114, etc., a higher accuracy may be achieved. For example wider internal channels may be connected to the fluid passages 24 extending from the first manifold 14, while narrower internal channels may be connected to fluid passages 124 extending from the second manifold 114. In this manner, the risk of pressure drops in one internal channel negatively affecting the flushing in another internal channel may be reduced.

In more detail, Fig. 5, illustrates an example of a channel irrigation system 110, in particular for flushing process medium through endoscopes having different width channels. The channel irrigation system 110 comprises a first flow producing device 12, a first manifold 14, a first pressure sensor 28, and a first plurality of fluid passages 24. The channel irrigation system 110 also comprises a second flow producing device 112, a second manifold 114, a second pressure sensor 128, and a second plurality of fluid passages 124. Similarly, to the example in Fig. 2, in the example illustrated in Fig. 5 alcohol and compressed air may be supplied to each one of the first and second manifolds 14,114 (as illustrated by the alcohol injectors 44, 144, the pressurized tanks 54, 154 and the actuator-controlled valves 52, 152). Furthermore, each one of the manifolds 14, 114 may be associated with an upstream flow sensor 40, 140 and a plurality of downstream flow sensors 42, 142. This dual setup of components may suitably be controlled by a common control unit 100. Furthermore, as explained previously, one and the same endoscope (or other medical article having multiple internal channels) may simultaneously be in fluid communication with both the first plurality of fluid passages 24 and the second plurality of fluid passages 124. Some of the internal channels of the medical article will be in fluid communication with the first plurality of fluid passages 24, while other internal channels of the medical article will be in fluid communication with the second plurality of fluid passages 124.

Since the drying stage is not as sensitive with respect to accuracy as, for instance, washing and disinfecting stages, it would be conceivable to, at least in some exemplary embodiments, supply the alcohol from a common alcohol injector to both manifolds 14, 114. Similarly, the compressed air may be supplied from a common pressurized tank to both manifolds 14, 114.

The channel irrigation system 110 illustrated in Fig. 5 further comprises redundancy pressure sensors 29, 129, one for each pressure manifold. Although not illustrated in the example of Fig. 2, it should be understood that also in that example, there also be provided a redundancy pressure sensor. Each redundancy pressure sensor 29, 129 is configured to measure the current pressure of the process medium inside the respective manifold 14, 114. The measurements are communicated to the control unit 100 similarly to the communication performed by the first pressure sensor 28 and the second pressure sensor 128. If, for instance, the control unit 100 determines that the value of the current pressure as provided by the first pressure sensor 28 deviates from the value provided by the associated redundancy pressure sensor 29 by at least a predefined amount, then the control unit 100 may issue an alert or alarm. Thus, incorrectly functioning pressure sensors may be quickly discovered and errors in the treatment of the medical article may be avoided.

This disclosure includes systems for supplying a process medium to medical articles, and methods of operating such systems. In particular, this disclosure includes systems and methods for enabling accurate processing, such as washing, cleaning, disinfecting and/or drying, of multiple internal channels of medical articles. This disclosure includes endoscope processing machinery, and extended systems including mutually compatible endoscope washer-disinfectors. It should be understood that various features disclosed herein are contemplated and disclosed in their various combinations and sub-combinations.

## Claims

1. A channel irrigation system for flushing process medium through internal channels of a medical article, in particular of an endoscope, comprising:
- a flow producing device configured to produce and supply a flow of a process medium,
- a manifold having an inlet and a plurality of outlets, the inlet being configured to receive the process medium supplied from the flow producing device,
- a plurality of fluid passages, each fluid passage having a first end connected to a respective one of said plurality of outlets for receiving process medium from the manifold, each fluid passage having a second end connectable to a respective internal channel of a medical article to be flushed with the process medium,
- a pressure sensor configured to measure the current pressure of the process medium inside the manifold,
- a control unit configured to receive from the pressure sensor information about the value of the current pressure and, based on the received information, control the operation of the flow producing device so that the process medium inside the manifold reaches a target pressure or target pressure range.

2. The channel irrigation system according to claim 1, wherein, upon determination by the control unit that the current pressure corresponds to said target pressure or target pressure range, the control unit is configured to control the operation of the flow producing device so that the pressure of the process medium inside the manifold is maintained at said target pressure or within said target pressure range.

3. The channel irrigation system according to any one of claims 1-2, wherein said controlling of the operation of the flow producing device includes the control unit controlling the speed of the flow producing device.

4. The channel irrigation system according to any one of claims 1-3, wherein said flow producing device is a gear pump.

5. The channel irrigation system according to any one of claims 1-4, further comprising:
- a conduit provided between the flow producing device and the manifold,
- an upstream flow sensor configured to measure the current flow rate in said conduit, and
- a plurality of downstream flow sensors, each downstream flow sensor being configured to measure the current flow rate in a respective one of said plurality of fluid passages.

6. The channel irrigation system according to claim 5,
wherein the control unit is configured to receive from the upstream flow sensor and the plurality of downstream flow sensors information about the value of the current flow rate in the conduit and the plurality of fluid passages, respectively,
wherein, upon determination by the control unit that the sum of the current flow rates of the plurality of fluid passages differs from the current flow rate in the conduit by at least a predefined amount, the control unit is configured to trigger an alert or alarm.

7. The channel irrigation system according to any one of claims 5-6,
wherein, for each one of said plurality of fluid passages, the control unit is configured to compare the value of the current flow rate with a respective nominal value,
wherein, upon determination by the control unit that the value of the current flow rate in any one of said plurality of fluid passages, deviates from its respective nominal value by at least a predetermined amount, the control unit is configured to trigger an alert or alarm.

8. The channel irrigation system according to any one of claims 5-7, wherein at least one of said upstream flow sensor and said plurality of downstream flow sensors is in the form of a paddlewheel flow sensor.

9. The channel irrigation system according to any one of claims 1-8, further comprising an alcohol injector configured to inject alcohol into the manifold to be further distributed into said plurality of fluid passages via said outlets of the manifold.

10. The channel irrigation system according to claim 9, wherein the control unit is configured to control the execution of one or more initial treatment stages in which the flow producing device is controlled to provide the process medium to the plurality of fluid passages via the manifold, and subsequently control the execution of one or more final treatment stages in which the alcohol injector is controlled to inject alcohol into the manifold.

11. The channel irrigation system according to any one of claims 1-10, wherein said pressure sensor is a first pressure sensor, wherein the channel irrigation system further comprises a redundancy pressure sensor configured to measure the current pressure of the process medium inside the manifold, wherein upon determination by the control unit that the respective values of the current pressure provided by the first pressure sensor and the redundancy pressure sensor deviate from each other by at least a predefined amount, the control unit is configured to issue an alert or alarm.

12. The channel irrigation system according to any one of claims 1-11, in particular for simultaneously flushing process medium through endoscope channels having different widths, wherein said flow producing device is a first flow producing device, said manifold is a first manifold, said plurality of fluid passages is a first plurality of fluid passages, and said pressure sensor is a first pressure sensor, the channel irrigation system further comprising:
- a second flow producing device configured to produce and supply a flow of a process medium,
- a second manifold having an inlet and a plurality of outlets, the inlet being configured to receive the process medium supplied from the second flow producing device,
- a second plurality of fluid passages, each fluid passage having a first end connected to a respective one of said plurality of outlets of the second manifold for receiving process medium from the second manifold, each fluid passage having a second end connectable to a respective internal channel of a medical article to be flushed with the process medium,
- a second pressure sensor configured to measure the current pressure of the process medium inside the second manifold,
wherein the control unit is configured to receive from the second pressure sensor information about the value of the current pressure and, based on the received information, control the operation of the second flow producing device so that the process medium inside the second manifold reaches a target pressure or target pressure range.

13. The channel irrigation system according to any one of the preceding claims, further comprising at least one paddlewheel flow sensor configured to measure a current flow rate of the process medium.

14. An endoscope washer-disinfector comprising the channel irrigation system according to any of the preceding claims, the endoscope washer-disinfector being configured for flushing the process medium through internal channels of one or more endoscopes using the channel irrigation system for cleaning them.
